# EUROPEAN PATENT SPECIFICATION

(11) **EP 4 234 466 B1**
(45) Date of publication and mention of the grant of the patent: **05.11.2025**
(21) Application number: 23155511.1
(22) Date of filing: 08.02.2023
(51) Int. Cl.: B65H 63/06, G01N 21/89, G01N 21/952, G01N 33/36

(54) **YARN MONITORING DEVICE**
GARNÜBERWACHUNGSVORRICHTUNG
DISPOSITIF DE SURVEILLANCE DE FIL

(30) Priority: 17.02.2022 JP 2022023163
(43) Date of publication of application: 30.08.2023
(73) Proprietor: Murata Machinery, Ltd., Kyoto-shi, Kyoto 601-8326 (JP)
(72) Inventor: Okajima, Kazuho, Kyoto-shi, Kyoto 612-8686 (JP)
(74) Representative: Hoffmann Eitle

(56) References cited:
- EP-B1- 0 244 788
- EP-B1- 0 677 731
- WO-A1-02/099400
- JP-A- 2018 177 380
- JP-A- 2019 128 477

## Description

### BACKGROUND OF THE INVENTION

### 1. Field of the Invention

The present invention relates to a yarn monitoring device and a yarn winding machine.

### 2. Description of the Related Art

A yarn monitoring device that detects a state of a yarn is mounted in a conventional yarn winding machine. For example, as explained in Japanese Patent Application Laid-Open No. 2018-177380, a yarn monitoring device that includes a first holder and a second holder that are stacked so as to be arranged in a running direction of the yarn is known in the art. The first holder constitutes a first detection module that has a function to detect a foreign substance contained in the yarn. The second holder constitutes a second detection module that has function to detect a thickness of the yarn. Each of the first detection module and the second detection module includes a light emitting element and a light receiving element. A state of the yarn is detected based on an amount of light received by the light receiving element. A light receiving window is provided in each of the first holder and the second holder. In the yarn monitoring device explained in Japanese Patent Application Laid-Open No. 2018-177380, each light receiving element is provided so as to be in a tight contact with each light receiving window.

Further, WO 02/099400 A1 is concerned with a pin hole detector. JP 2019 128477 A is concerned with a light control body. EP 0 677 731 B1 is concerned with an optical sensor device.

### Problem to be solved by the Invention

A running area of a yarn is in a form of a groove having a shape of English letter U that opens on a side on which a working passage is provided with respect to the yarn winding machine (front side). In other words, an open portion is formed on the front side of the running area of the yarn. An inlet and an outlet of the yarn are formed at an upstream side and a downstream side of the running area of the yarn, respectively. In the yarn monitoring device, a disturbance light that may enter the running area via the open portion, the inlet, or the outlet may affect the amount of the light received.

### SUMMARY OF THE INVENTION

One object of the present invention is to provide a yarn monitoring device and a yarn winding machine capable of reducing an effect of a disturbance light.

A yarn monitoring device according to one aspect of the present invention includes a casing; a detecting section; and a regulating section. The casing includes a first member and a second member having surfaces thereof facing each other in a first direction that is orthogonal to a running direction of a yarn. A running area of the yarn is formed between the first member and the second member, and an open portion that opens toward a second direction that is orthogonal to the running direction and the first direction, and opens toward the running direction is formed. The detecting section detects the yarn that is running inside the running area. The detecting section includes at least one light emitting element provided in at least one of the first member and the second member, and at least one light receiving element provided in at least one of the first member and the second member and including a light receiving surface that is arranged facing an opening formed on the first member and / or the second member. The regulating section includes a plurality of light shielding surface sections that forms an angle within a range of 0 degrees to 90 degrees, including both the values, on an opposite side of the open portion with respect to the light receiving surface, and a passing section that is formed between the light shielding surface sections and allows passage of a transmitted light passed through the yarn running inside the running area or a reflected light reflected by the yarn. The regulating section is provided in at least one location among inside the opening, between the opening and the light receiving surface, and on the running area side of the opening.

The above and other objects, features, advantages and technical and industrial significance of this invention will be better understood by reading the following detailed description of presently preferred embodiments of the invention, when considered in connection with the accompanying drawings.

### BRIEF DESCRIPTION OF THE DRAWINGS

FIG. 1 is a front view of a spinning machine that is a yarn winding machine according to a first embodiment.
FIG. 2 is a perspective view of a yarn monitoring device of the spinning machine shown in FIG. 1.
FIG. 3 is a perspective view of an internal structure of the yarn monitoring device shown in FIG. 2.
FIG. 4 is a perspective view of a holder coupler shown in FIG. 3.
FIG. 5 is a plan cross-sectional view of a first holder shown in FIG. 4.
FIG. 6A is view showing a configuration example of a light shielding surface section of a regulating section shown in FIG. 5.
FIGS. 6B and 6C are views showing other configuration examples of the light shielding surface section.
FIG. 7 is a plan cross-sectional view of a second holder shown in FIG. 4.
FIG. 8 is a perspective view showing overlapping of the regulating section and a second regulating section.
FIG. 9 is a plan cross-sectional view of a second holder according to a modification.
FIG. 10 is a disassembled perspective view of a dust-proof structure in the second holder shown in FIG. 9.
FIG. 11 is a cross-sectional view along a XI-XI line shown in FIG. 9.
FIG. 12 is a cross-sectional view along a XII-XII line shown in FIG. 11.
FIG. 13 is a view showing dimensions and arrangement of a light receiving element included in the second holder shown in FIG. 9.

### DETAILED DESCRIPTION

Exemplary embodiments of the present invention are explained below in detail with reference to the accompanying drawings. Identical components or corresponding components are indicated by the same reference symbols in the drawings and redundant explanation thereof is omitted.

### Structure of Spinning Machine

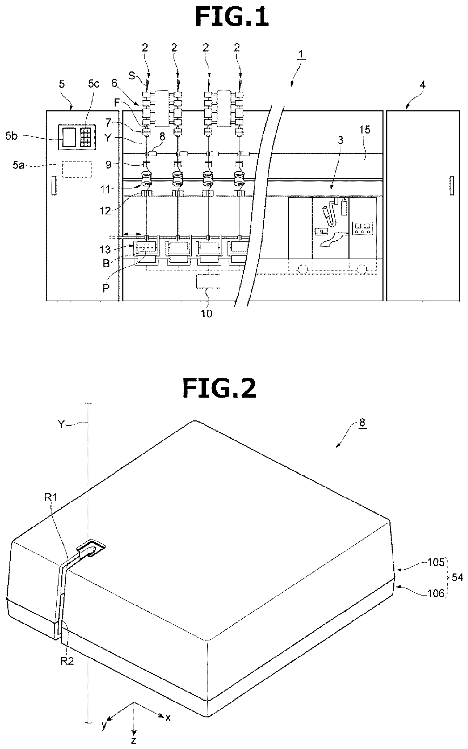

As shown in FIG. 1, the spinning machine 1 includes a plurality of spinning units 2, a yarn joining cart 3, a not-shown doffing cart, a first-end frame 4, and a second-end frame 5. The spinning units 2 are arranged side-by-side. Each of the spinning units 2 generates a yarn Y and winds the yarn Y into a package P. The yarn joining cart 3 performs a yarn joining operation in a certain spinning unit 2 in which the yarn Y is cut or becomes discontinuous for some reason. When the package P becomes fully wound in a certain spinning unit 2, the doffing cart doffs the fully wound package P and supplies a new bobbin B to that spinning unit 2.

Devices such as a collecting device for collecting fiber waste, yarn waste, and the like produced in the spinning unit 2, are accommodated in the first-end frame 4. An air supplying section that supplies compressed air (air) to various parts of the spinning machine 1 after appropriately adjusting an air pressure of the compressed air, and a driving motor and the like that supplies driving power to various elements of the spinning unit 2 are arranged in the second-end frame 5. The second-end frame 5 is provided with a machine-frame controlling device 5a, a display screen 5b, and one or more input keys 5c. The machine-frame controlling device 5a centrally manages and controls various elements of the spinning machine 1. The display screen 5b displays information and the like about setting contents and / or a state of the spinning unit 2. An operator can perform various settings of the spinning unit 2 by appropriately operating the input keys 5c.

Each of the spinning units 2 includes a drafting device 6, an air spinning device 7, the yarn monitoring device 8, a tension sensor 9, a yarn accumulating device 11, a waxing device 12, and a winding device 13 in this order from upstream in a running direction of the yarn Y. One unit controller 10 is arranged for a predetermined number of the spinning units 2, and the unit controller 10 controls operations of those spinning units 2. In each spinning unit 2, the drafting device 6 and the air spinning device 7 function as a yarn supplying device that supplies the yarn Y.

The drafting device 6 drafts a sliver (fiber bundle) S. The air spinning device 7 generates the yarn Y by applying twists to a fiber bundle F, drafted by the drafting device 6, by using a swirling air current. The yarn accumulating device 11 removes a slack of the yarn Y at a location between the air spinning device 7 and the winding device 13. The waxing device 12 applies wax to the yarn Y at a location between the yarn accumulating device 11 and the winding device 13. The winding device 13 winds the yarn Y supplied from the drafting device 6 and the air spinning device 7, which collectively function as a yarn supplying device, around the bobbin B thereby forming the package P.

The yarn monitoring device 8 monitors, at a location between the air spinning device 7 and the yarn accumulating device 11, information about the running yarn Y, and detects presence / absence of a yarn defect in the yarn Y based on the monitored information. Upon detecting the yarn defect, the yarn monitoring device 8 transmits a yarn-defect detection signal to the unit controller 10. The yarn monitoring device 8 detects, for example, a thickness abnormality of the yarn Y and / or a foreign substance contained in the yarn Y as the yarn defect. The yarn monitoring device 8 also detects a yarn breakage and the like. The tension sensor 9 measures, at a location between the air spinning device 7 and the yarn accumulating device 11, a tension of the running yarn Y, and transmits a tension measurement signal representing the measured tension to the unit controller 10. When the unit controller 10 determines, based on a detection result obtained in the yarn monitoring device 8 and / or the tension sensor 9, that an abnormality is present in the yarn Y, the yarn Y is cut in that spinning unit 2.

In the following explanation, a side on which a working passage is provided with respect to the spinning machine 1, or a side on which a yarn path is positioned with respect to the yarn joining carrier 3 is referred to as a "front side (machine front side)" and an opposite side thereof is referred to as a "rear side (machine rear side)". The working passage is used by the operator to access various parts of the spinning machine 1. Terms "up" and "down" correspond to an upward direction and a downward direction in a vertical direction, respectively.

### Configuration of Yarn Monitoring Device

A yarn monitoring device 8 according to a first embodiment will be explained below with reference to FIG. 2 onwards. In FIGS. 2 to 8 the xyz orthogonal coordinate system is shown as a reference of a position, arrangement, or orientation of members or components.

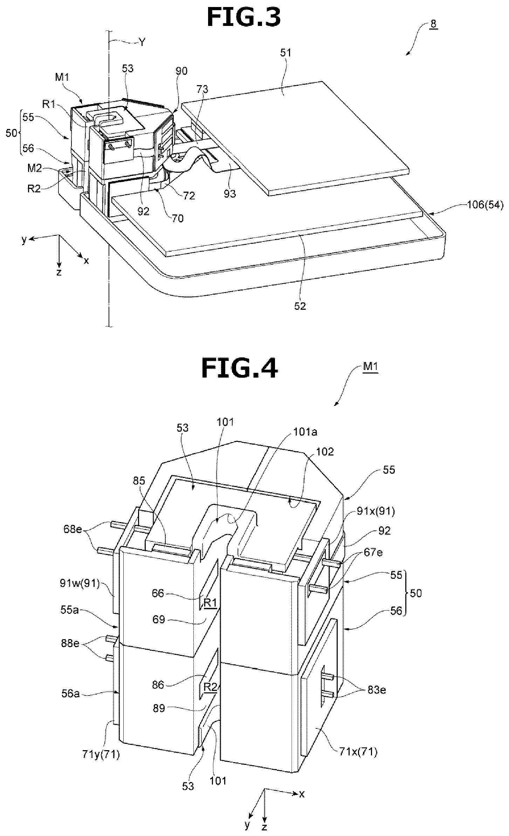

As shown in FIGS. 2 and 3, the yarn monitoring device 8 includes a holder coupler 50, a first flat substrate 51, a second flat substrate 52, a yarn path guide 53, and a case 54. The holder coupler 50 includes a first holder 55 and a second holder 56 that are stacked in the running direction of the yarn Y. The running direction of the yarn Y is parallel to the z direction shown in the drawings.

As shown in FIGS. 3 to 5, the first holder 55 constitutes a first detection module M1 through which the running yarn Y is passed to detect a state of the yarn Y. The first detection module M1 includes at least a foreign substance detection function for detecting a foreign substance contained in the running yarn Y. The first holder 55 is arranged on an upstream side of the second holder 56. The first holder 55 includes a casing 55a that is made of, for example, resin. A running area R1 is provided in the casing 55a of the first holder 55. The running area R1 is a space that extends along a running path of the yarn Y and opens on the front side. The running area R1 is a passage through which the running yarn Y passes, and it is formed such that it extends from upstream to downstream and is a groove having a shape of English letter U that is open on the front side. The running area R1 communicates with a running area R2 of the second holder 56. An upper surface of the casing 55a of the first holder 55 includes an upstream side concave part 102, which is a space in which the yarn path guide 53 is provided.

Light emitting elements 63, 64 and light receiving elements 67, 68, which measure the state of the yarn Y that is running through the running area R1, are mounted inside the casing 55a of the first holder 55. The first holder 55 supports the light emitting elements 63, 64 and the light receiving elements 67, 68. The light emitting elements 63, 64 and the light receiving elements 67, 68 are arranged such that the optical axes thereof are positioned on the same plane that is orthogonal to the running direction of the yarn Y. A light emitting diode (LED) and the like, for example, can be used as the light emitting elements 63, 64. A photodiode and the like, for example, can be used as the light receiving elements 67, 68.

The light emitting element (second light emitting element) 64 includes a lead pin 64e and the light emitting element (first light emitting element) 63 includes a lead pin 63e. Tips of the lead pins 64e and 63e extend away from the running area R1. The light receiving element (second light receiving element) 68 includes a pair of lead pins 68e, and the light receiving element 67 (first light receiving element) includes a pair of lead pins 67e.

The first detection module M1 irradiates a light from the light emitting element 64 onto the yarn Y that is running through the running area R1. A reflected light, which is light reflected by the yarn Y among the light emitted from the light emitting element 64, is received by the light receiving element 68. A transmitted light, which is a light that passes through the yarn Y among the light emitted from the light emitting element 64, is received by the light receiving element 67. On the other hand, the first detection module M1 irradiates a light from the light emitting element 63 onto the yarn Y that is running through the running area R1. A reflected light, which is a light reflected by the yarn Y among the light emitted from the light emitting element 63, is received by the light receiving element 67. A transmitted light, which is a light that passes through the yarn Y among the light emitted from the light emitting element 63, is received by the light receiving element 68. A foreign substance contained in the yarn Y is detected based on an amount of the reflected light received by each of the light receiving elements 68 and 67.

The first holder 55 includes a rigid flexible substrate 90. The rigid flexible substrate 90 includes a plurality of rigid parts 91, a plurality of flexible parts 92, and a flexible connecting part 93. The rigid parts 91 are made of material having high rigidity such as glass epoxy. The flexible parts 92 and the flexible connecting part 93 are made of material having high flexibility and high bendability such as polyimide.

A rigid part 91 is a rigid substrate provided with a wiring pattern, through holes, and the like. A plurality of the rigid parts 91 is fixed with one or more screws to side surfaces of the second holder 56. The lead pins 68e of the light receiving element 68 are connected, by soldering and the like, to a rigid part 91w on the light receiving element 68 side among the rigid parts 91. The lead pins 67e of the light receiving element 67 are connected, by soldering and the like, to a rigid part 91x on the light receiving element 67 side among the rigid parts 91. The lead pins 64e of the light emitting element 64 are connected, by soldering and the like, to a rigid part 91y on the light emitting element 64 side among the rigid parts 91. The lead pins 63e of the light emitting element 63 are connected, by soldering and the like, to a rigid part 91z on the light emitting element 63 side among the rigid parts 91. The flexible part 92 is a flexible substrate provided with a wiring pattern and the like. The flexible part 92 electrically connects the rigid parts 91 that are adjacent to each other. The flexible connecting part 93 electrically connects the light emitting elements 64, 63 and the light receiving elements 68, 67, which are mounted on the first holder 55, to the first flat substrate 51.

As shown in FIGS. 3, 4, and 7, the second holder 56 constitutes a second detection module M2 through which the running yarn Y is passed to detect the state of the yarn Y. The second detection module M2 has a yarn thickness detection function to detect a thickness of the running yarn Y. The second holder 56 is arranged on the downstream side of the first holder 55. The second holder 56 includes a casing 56a that is made of, for example, resin. The running area R2 is provided in the casing 56a of the second holder 56. The running area R2 is a space that extends along a running path of the yarn Y and opens on the front side. The running area R2 is a passage through which the running yarn Y passes, and it is formed such that it extends from upstream to downstream and is a groove having a shape of English letter U that is open on the front side.

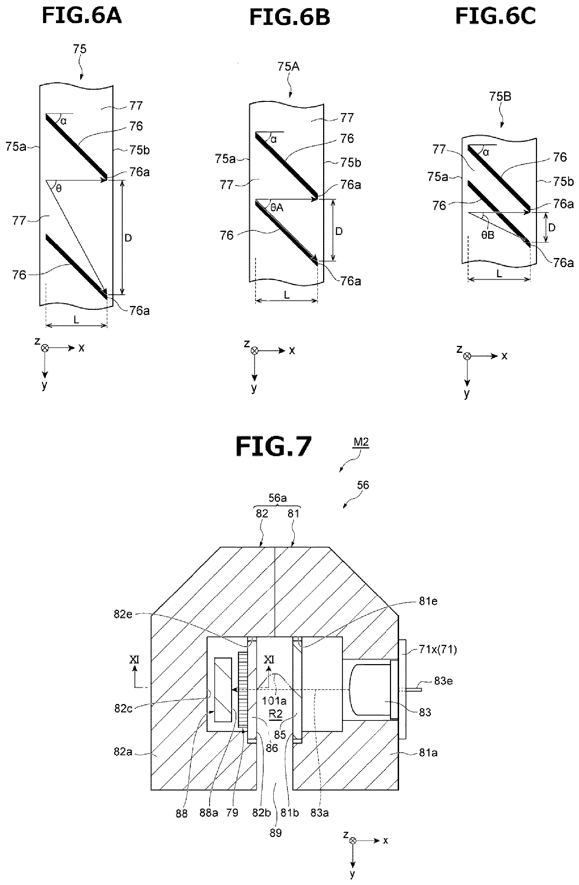

A light emitting element 83 and a light receiving element 88 that measure the state of the yarn Y that is running through the running area R2 are mounted inside the casing 56a of the second holder 56. The second holder 56 supports the light emitting element 83 and the light receiving element 88. The light emitting element 83 and the light receiving element 88 are arranged across the running area R2 facing each other. An LED and the like, for example, can be used as the light emitting element 83. A photodiode and the like, for example, can be used as the light receiving element 88. The light emitting element 83 has lead pins 83e. Tips of the lead pins 83e extend away from the running area R1. The light receiving element 88 has a pair of lead pins 88e. Note that a later-explained flexible part is not shown in FIG. 7.

The second detection module M2 irradiates light from the light emitting element 83 onto the yarn Y that is running through the running area R2. A transmitted light, which is a light passes through the yarn Y among the light emitted from the light emitting element 83, is received by the light receiving element 88. The thickness of the yarn Y is detected based on the amount of the transmitted light received by the light receiving element 88.

The second holder 56 includes a rigid flexible substrate 70. The rigid flexible substrate 70 includes a plurality of rigid parts 71, a plurality of flexible parts 72, and a flexible connecting member 73. Rigid material such as glass epoxy is used for the rigid parts 71. the flexible parts 72 and the flexible connecting part 73 are made of a material having high flexibility and bendability such as polyimide.

A rigid part 71 is a rigid substrate provided with a wiring pattern, through holes, and the like. A plurality of the rigid parts 71 is fixed with one or more screws to side surfaces of the second holder 56. The lead pins 83e of the light emitting element 83 are connected, by soldering and the like, to a rigid part 71x on the light emitting element 83 side among the rigid parts 71. The lead pins 88e of the light receiving element 88 are connected, by soldering and the like, to a rigid part 71y on the light receiving element 88 side among the rigid parts 71. The flexible part 72 is a flexible substrate provided with a wiring pattern and the like. The flexible part 72 electrically connects the rigid parts 71 that are adjacent to each other. The flexible connecting member 73 electrically connects the light emitting element 83 and the light receiving element 88, which are mounted on the second holder 56, to a second flat substrate 52. One end of the flexible connecting member 73 is electrically connected to a rigid part 71 arranged on a back side of the second holder 56.

The first flat substrate 51 shown in FIG. 3 processes the signals to be input and output to the light emitting elements 64, 63 mounted on the first holder 55. The second flat substrate 52 processes the signals to be input and output to the light emitting element 83 mounted on the second holder 56.

As shown in FIGS. 3 and 4, the yarn path guides 53 regulate the running path of the yarn Y and guide the running yarn Y. Each of the yarn path guides 53 is arranged inside the case 54 on each of upstream and downstream sides of the holder coupler 50. One yarn path guide 53 is arranged in the upstream side concave part 102 of the first holder 55. The other yarn path guide (not shown) is arranged in a not-shown downstream side concave part of the second holder 56. The yarn path guides 53 are formed of a material (for example, ceramic or titanium and the like) having resistance to wear, and has a shape of a rectangular plate. The yarn path guide 53 includes a grooved section 101 having a bottom 101a form in a shape of English letter U or V. The yarn path guide 53 guides the yarn Y through the grooved section 101 to the running area R1, R2.

As shown in FIGS. 2 and 3, the case 54 constitutes an outer part of the yarn monitoring device 8. The case 54 includes an upper casing 105 arranged upstream and a lower casing 106 arranged downstream. The upper casing 105 and the lower casing 106 can be coupled to each other with one or more screws. The case 54 accommodates the holder coupler 50, the first flat substrate 51, the second flat substrate 52, and the yarn path guide 53.

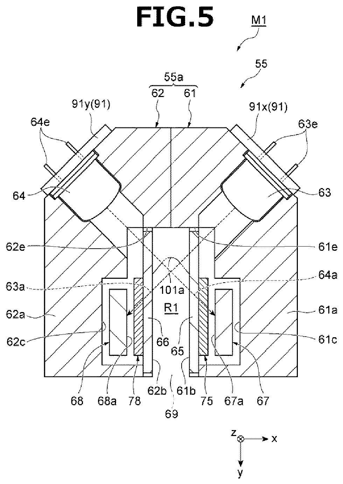

Configuration of the first holder 55 and the second holder 56 in the yarn monitoring device 8 will be explained in detail with reference to FIGS. 5 to 8. First, the first holder 55 will be explained with reference to FIGS. 5, 6A, 6B, and 6C. The casing 55a of the first holder 55 includes a first member 61 and a second member 62 that have surfaces thereof facing each other in the x direction (first direction) that is orthogonal to the running direction of the yarn Y. In other words, the casing 55a can be divided in the x direction. The casing 55a includes a pair of separable parts that is a combination of a left part and a right part. However, the first holder 55 can be an integral body that cannot be separated into the left part and the right part. Even in such a configuration, the first member 61 and the second member 62 are arranged facing each other in the x direction.

An opposite surface 61b and a housing space 61c are formed on a front section 61a of the first member 61. The opposite surface 61b extends along a yz direction. A rectangular opening (first opening) 61e that communicates with the housing space 61c, for example, is formed on the opposite surface 61b. A light receiving window 65 that is transparent to the light emitted from the light emitting element 64 (visible light) is fixed inside the opening 61e. The light emitting element 63 explained above is provided inside the first member 61. The light receiving element 67 explained above is arranged and fixed in the housing space 61c. The light receiving surface (first light receiving surface) 67a of the light receiving element 67, for example, extends parallel to a yz plane and is arranged facing the opening 61e (the light receiving window 65 in the present embodiment).

On the other hand, an opposite surface 62b and a housing space 62c are formed on a front section 62a of the second member 62. The opposite surface 62b extends along the yz direction. A rectangular opening (second opening) 62e that communicates with the housing space 62c, for example, is formed on the opposite surface 62b. A light receiving window 66 that is transparent to the light emitted from the light emitting element 63 (visible light) is fixed inside the opening 62e. The light emitting element 64 explained above is provided inside the second member 62. The light receiving element 68 explained above is arranged and fixed in the housing space 62c. The light receiving surface (second light receiving surface) 68a of the light receiving element 68, for example, extends parallel to the yz plane and is arranged facing the opening 62e (the light receiving window 66 in the present embodiment).

The opposite surface 61b and the opposite surface 62b are separated by a predetermined distance in the x direction, and the running area R1 of the yarn Y is formed between the opposite surface 61b and opposite surface 62b. An open portion 69 that opens to the y direction (second direction orthogonal to the running direction and the first direction) is formed in the running area R1. The open portion 69 penetrates the first holder 55 in the z direction. In other words, the open portion, too, opens in the z direction (running direction).

In the first detection module M1, the light emitting element 63, the light emitting element 64, the light receiving element 67, and the light receiving element 68 constitute a detecting section that detects foreign matter contained in the yarn Y (state of the yarn Y). An optical axis 63a of the light emitting element 63 is inclined at a predetermined angle (for example, 45 degrees) with respect to a xz plane. An optical axis 64a of the light emitting element 64 is inclined at a predetermined angle (for example, 45 degrees) with respect to the xz plane. The optical axis 63a and the optical axis 64a intersect near the front section of the bottom 101a of the grooved section 101. In the present specification, when referring to the phrase "the bottom 101a of the grooved section 101" for explaining a position or an arrangement of the components of the first detection module M1 or the second detection module M2, the position or the arrangement of "the bottom 101a of the grooved section 101" refers to the position or arrangement of the grooved section 101 of the yarn path guides 53 projected in the cross-sectional views shown in FIGS. 5 and 7 in the z direction. Therefore, in FIGS. 5 and 7, the "grooved section 101" and the "bottom 101a" are indicated by virtual lines. The position of the "bottom 101a" is roughly synonymous with the position of the yarn path along which the yarn Y runs.

A regulating section (first regulating section) 75 for reducing incidence of a disturbance light is provided between the opening 61e and the light receiving surface 67a of the light receiving element 67, in other words, between the light receiving window 65 and the light receiving surface 67a. The disturbance light is a light coming from a direction that is different from the direction from which the transmitted light from the light emitting element 64 is coming and the reflected light from the light emitting element 63 is coming. The regulating section 75 regulates the direction of the light received by the light receiving element 67. The regulating section 75 extends parallel to the yz plane.

Configuration of the regulating section 75 is explained in detail with reference to FIG. 6A. As shown in FIG. 6A, the regulating section 75 is, for example, a plate-shaped member that includes a plurality of light shielding surface sections 76 arranged parallel to each other and a transparent section (passing section) 77 that is formed between the light shielding surface sections 76. The light shielding surface sections 76 are made of, for example, black silicone rubber with high light shielding properties, and exhibit a louver structure. The transparent section 77, for example, is made of a transparent silicone rubber. Alternatively, a film made of polycarbonate and the like can be provided on a front surface 75a and a back surface 75b of the regulating section 75. Each light shielding surface section 76 inclines at a predetermined angle with respect to a xz plane. Each light shielding surface section 76 forms an angle of 90 degrees or less on an opposite side of the open portion 69 in the y direction with respect to the light receiving surface 67a of the light receiving element 67. An inclination angle of the light shielding surface sections 76 with respect to the light receiving surface 67a is an acute angle. The inclination angle of the light shielding surface sections 76 is, for example, equal to the inclination angle of the optical axis 64a of the light emitting element 64 with respect to the yz plane. Each light shielding surface section 76 extends in a direction that intersects the y direction (extending direction). Moreover, each light shielding surface section 76 also has a predetermined length in the z direction (that is, it also extends in the z direction).

When seen from another perspective, the extending direction of each light shielding surface section 76 of the regulating section 75 is the same as a direction that connects the light emitting element 64 and the light receiving element 67. It is sufficient that the extending direction of each light shielding surface section 76 of the regulating section 75 forms an angle of ±5 degrees or less, preferably ±3 degrees or less, with respect to the direction (first connecting direction) that connects the light emitting element 64 and the light receiving element 67. In the present specification, a positive (+) or a negative (-) sign may be used to indicate angles. The positive angle is an angle formed on the bottom 101a side of the grooved section 101, and the negative angle is an angle formed on an open portion 89 side.

Each light shielding surface section 76 of the regulating section 75 has a length L in the x direction. The two adjacent light shielding surface sections 76 are arranged at a distance D in the y direction (not the distance in a direction that is orthogonal to the light shielding surface sections 76.). When the angle between the extending direction of the light shielding surface sections 76 and the x direction (first direction) is an angle α (α is an angle of 90 degrees or less), a relation between the length L and the distance D, that is, distance D/length L > tan α holds true. The relation between the length L and the distance D, that is, distance D/length L > 1 holds true. The relation between the length L and the distance D is 3 ≥ distance D/length L > 1 holds true. If distance D/length L is 3, an angle θ of the triangle determined by the length L and the distance D is approximately 72 degrees.

A regulating section (second regulating section) 78 for reducing incidence of a disturbance light is provided between the opening 62e and the light receiving surface 68a of the light receiving element 68, in other words, between the light receiving window 66 and the light receiving surface 68a. The disturbance light is a light coming from a direction that is different from the direction from which the transmitted light from the light emitting element 63 is coming and the reflected light from the light emitting element 64 is coming. The regulating section 78 provided on the second member 62 arranged facing the first member 61 has the same configuration as that of the regulating portion 75. The regulating section 78 regulates the direction of the light received by the light receiving element 68. The regulating section 78 has a structure that is plane-symmetrical with the regulating section 75 with respect to a plane parallel to the yz plane and a plan that passes through the bottom 101a (or the divided plane of the first member 61 and the second member 62). Each light shielding surface section of the regulating section 78 forms an angle of 90 degrees or less on an opposite side of the open portion 69 in the y direction with respect to the light receiving surface 68a of the light receiving element 68. An inclination angle of the light shielding surface sections with respect to the light receiving surface 68a is an acute angle. The inclination angle of the light shielding surface sections of the regulating section 78 is, for example, equal to the inclination angle of the optical axis 63a of the light emitting element 63 with respect to the yz plane. Each light shielding surface section extends in a direction that intersects the y direction (extending direction).

When seen from another perspective, the extending direction of each light shielding surface section of the regulating section 78 is the same as the direction (second connecting direction) that connects the light emitting element 63 and the light receiving element 68. It is sufficient that the extending direction of each light shielding surface section of the regulating section 78 forms an angle of ±5 degrees or less, preferably ±3 degrees or less, with respect to the direction that connects the light emitting element 63 and the light receiving element 68. In the regulating section 75 and the regulating section 78 shown in FIG. 5, a direction of the hatched lines is in alignment with the extending direction of the light shielding surface sections.

A configuration in which the relation explained above is not established between the length L and the distance D will be explained below. As shown in FIG. 6B, when the ratio of distance D/length L is 1, a regulating section 75A can allow the transmitted light to pass through, but is unlikely to allow the reflected light to pass through. An angle θA in such a configuration is 45 degrees. In such a configuration, the angle θA is equal to angle α, and the relation between the distance (D) and the length (L), that is, distance D/length L = tan α holds true. As shown in FIG. 6C, if the ratio of the distance D/length L is less than 1, the regulating section 75B can allow transmitted light to pass through, but cannot allow the reflected light to pass through. The angle θB in such a configuration is less than 45 degrees. Therefore, it is suitable for the first detection module M1 to have the above relation (3 ≥ distance D/length L > 1) established in the regulating section 75. Alternatively, it is suitable for the first detection module M1 to have the above relation (distance D/length L > tan α) established in the regulating section 75. In such a configuration, it is advantageous to control the length L than to control the distance D. It is preferable that the above relation be satisfied by adjusting the length L.

The second holder 56 will be explained with reference to FIG. 7. The casing 56a of the second holder 56 includes a first member 81 and a second member 82 that have surfaces thereof facing each other in the x direction (first direction) that is orthogonal to the running direction of the yarn Y. In other words, the casing 56a can be divided in the x direction. The casing 56a includes a pair of separable parts that is a combination of the left part and the right part. However, the second holder 56 can be an integral body that cannot be separated into the left part and the right part. Even in such a configuration, the first member 81 and the second member 82 are arranged facing each other in the x direction.

An opposite surface 81b is formed on a front section 81a of the first member 81. The opposite surface 81b extends along the yz direction. A rectangular opening 81e, for example, is formed on the opposite surface 81b. A window section 85 that is transparent to the light emitted from the light emitting element 83 (visible light) is fixed inside the opening 81e. The light emitting element 83 explained above is provided inside the first member 81.

On the other hand, an opposite surface 82b and a housing space 82c are formed on a front section 82a of the second member 82. The opposite surface 82b extends along the yz direction. A rectangular opening 82e that communicates with the housing space 82c, for example, is formed on the opposite surface 82b. A light receiving window 86 transparent to the light emitted from the light emitting element 83 (visible light) is fixed inside the opening 82e. The light receiving element 88 explained above is arranged and fixed in the housing space 82c. The light receiving surface 88a of the light receiving element 88, for example, extends parallel to the yz plane, and faces toward the opening 82e (the light receiving window 86 in the present embodiment).

The opposite surface 81b and the opposite surface 82b are separated by a predetermined distance in the x direction, and the running area R2 of the yarn Y is formed between the opposite surface 81b and opposite surface 82b. The open portion 89 that opens to the y direction (second direction orthogonal to the running direction and the first direction) is formed in the running area R2. The open portion 89 penetrates the second holder 56 in the z direction. In other words, the open portion, too, opens in the z direction (running direction).

In the second detection module M2, the light emitting element 83 and the light receiving element 88 constitute a detecting section that detects the thickness of the yarn Y (state of yarn Y). An optical axis 83a of the light emitting element 83, for example, is parallel to the x direction.

A regulating section 79 for reducing incidence of the disturbance light from a different direction than the transmitted light from the light emitting element 83 is provided between the opening 81e and the light receiving surface 88a of the light receiving element 88, in other words, between the light receiving window 86 and the light receiving surface 88a. The regulating section 79 extends parallel to the yz plane. Other than the orientation of the light shielding surface section, the regulating section 79 has the same configuration as that of the regulating section 75 of the first detection module M1. Each light shielding surface section of the regulating section 79 is, for example, parallel to the optical axis 83a of the light emitting element 83. In other words, each light shielding surface section of the regulating section 79 is orthogonal to the y direction. Each light shielding surface section of the regulating section 79 forms an angle of 90 degrees or less on an opposite side of the open portion 89 in the y direction with respect to the light receiving surface 88a of the light receiving element 88 (in such a configuration, an angle on the open portion 89 side, too, is 90 degrees). Each light shielding surface section of the regulating section 79 extends in a direction that intersects the y direction (extending direction).

When seen from another perspective, the extending direction of each light shielding surface section of the regulating section 79 is the same as a direction that connects the light emitting element 83 and the light receiving element 88. It is sufficient that the extending direction of each light shielding surface section of the regulating section 79 forms an angle of ±5 degrees or less, preferably ±3 degrees or less, with respect to the direction that connects the light emitting element 83 and the light receiving element 88. In the regulating section 79 shown in FIG. 7, a direction of the hatched lines aligns with the extending direction of the light shielding surface sections.

In the regulating section 79, the relation between the length L and the distance D is distance D/length L ≤ 1 holds true.

According to the yarn monitoring device 8 and the spinning machine 1 according to the present embodiment, the light receiving surfaces 67a, 68a, 88a of the light receiving elements 67, 68, 88 face the openings 61e, 62e, 82e formed in the first member 61 and the second members 62, 82. The transmitted light passed through the yarn Y or the reflected light reflected by the yarn Y is input to the light receiving elements 67, 68, and 88 through the openings 61e, 62e, 82e. The light input to the light receiving elements 67, 68, 88 reaches the light receiving surfaces 67a, 68a, 88a after passing through the transparent section 77 of the regulating section 75, 78, and 79. The regulating sections 75, 78, and 79 include the plurality of the light shielding surface sections 76 that forms an angle of 90 degrees or less on the opposite side of the open portion 69, 89 with respect to the light receiving surfaces 67a, 68a, and 88a. The plurality of the light shielding surface sections 76 are not parallel to the light receiving surfaces 67a, 68a, and 88a, and an acute or a right angle formed between the light receiving surfaces 67a, 68a, and 88a faces away from the open portions 69, 89. Accordingly, the plurality of the light shielding surface sections 76 allows the light in the transparent section 77 to pass while shielding the light from a predetermined direction (for example, light entering through the open portions 69, 89). According to the yarn monitoring device 8 that includes the regulating sections 75, 78, and 79, an effect of a disturbance light can be reduced.

In the first detection module M1 and the second detection module M2, the light shielding surface sections 76 of the regulating sections 75, 78, and 79 extend in a direction that intersects the y direction. Because the light shielding surface sections 76 are not parallel to the y direction, the light shielding surface sections 76 can shield the disturbance light coming from the y direction. The light that enters through the open portions 69, 89 can be shielded.

In the second detection module M2, the light emitting element 83 and the light receiving element 88 are arranged facing each other in the x direction, and the relation between the distance (D) and the length (L), that is, "distance (D)/length (L) ≤ 1" holds true. The distance (D) is less than the length (L). Accordingly, the disturbance light from the y direction can be certainly shielded in a yarn monitoring device 8 in which the light emitting element 83 and the light receiving element 88 are arranged facing each other in the x direction.

In the first detection module M1, the first member 61 includes the light receiving element 67 and the light emitting element (first light emitting element) 63, and the second member 62 includes the light emitting element (second light emitting element) 64. In the regulating section 75, the relation between the distance (D) and the length (L), that is, "distance (D)/length (L) ≥ 1" holds true. The distance (D) is equal to or higher than the length (L). Accordingly, the transmitted light can be detected by the light emitting element 64 and the reflected light can be detected by the light emitting element 63. By establishing the relation explained above, detection of the reflected light by the light emitting element 63 is not hindered. In such a yarn monitoring device 8, an effect of a disturbance light can be reduced. The same effects and advantages can be achieved in the light receiving element 68, the light emitting element 64 that constitutes the first light emitting element, the light emitting element 63 that constitutes the second light emitting element, and the regulating section 78.

In the first detection module M1, the relation between the distance (D) and the length (L), that is, "distance (D)/length (L) ≤ 3" holds true. The distance (D) is set to shield the incident of the disturbance light while allowing the detection of the reflected light. Accordingly, noise in the detection signal is reduced, thus improving the S/N ratio.

Embodiments of the present invention are explained above; however, the present invention is not limited to the embodiments explained above. For example, at least one of the regulating sections 75, 78, and 79 can be provided on the running area (running area R1 or running area R2) side of the opening. At least one of the regulating sections 75, 78, and 79 can be provided inside the opening. In such a configuration, a light receiving window is omitted. It is acceptable if a member made of a transparent silicone rubber or other material is not provided between the light shielding surface sections in one or more among the regulating sections 75, 78, 79. In one or more among the regulating sections 75, 78, 79, a passing section, which is a space that allows the transmitted light passed through the yarn Y or the reflected light reflected by the yarn Y to pass through, can be provided between the light shielding surface sections.

In the first detection module M1 and the second detection module M2, one or more among the regulating sections 75, 78, 79 can be provided only on the open portion side of the opening. In such a configuration, the length of the regulating sections 75, 78, 79 in the y direction (second direction) can be half or less or approximately half of the regulating sections 75, 78, 79 according to the above embodiment. In other words, the regulating section is provided only in an approximately lower half of the regulating section 75, 78, 79 shown in FIGS. 5 and 7 or an area smaller than the lower half of the regulating section 75, 78, 79. Accordingly, the disturbance light can be reduced while preventing attenuation of light from the light source (the light emitting element).

The present invention can be applied to a yarn monitoring device in which a first member includes a light emitting element and a light receiving element, and the light emitting element is provided on an opposite side of an open portion in a y direction with respect to the light receiving element. In such a configuration, the extending direction of the light shielding surface section and the emitting direction of the light emitting element, for example, intersect at about 90 degrees. The extending direction of the light shielding surface section can form an angle of 90 ± 5 degrees with respect to the emission direction of the light emitting element. It is sufficient that the extending direction of the light shielding surface section forms an angle of 90 ± 3 degrees with respect to the emitting direction of the light emitting element. According to the present configuration, an effect of a disturbance light can be reduced in the yarn monitoring device that detects the reflected light without using the transmitted light. Even in such a configuration, a relation between the length L of each light shielding surface section in the x direction (first direction) and the distance D in the y direction between the two adjacent light shielding surface sections, that is, "1 < Distance D/Length L ≤ 3" holds true.

The light shielding surface section of the regulating section can extend in the y direction (second direction) as well as in the x direction (first direction). That is, the light shielding surface section of the regulating section can extend in the xy plane. Accordingly, the light that enters through an inlet and / or an outlet (open portion) of the yarn Y positioned in the running direction of the yarn inside the casing can be shielded. The light shielding surface section of the regulating section can extend such that it makes an angle of 5 degrees or less with respect to the x direction.

A relation between the length L of each light shielding surface section in the x direction (first direction) and the distance D in the y direction between the two adjacent light shielding surface sections, that is, "distance D/length L ≤ 1" may hold true. Moreover, in the second detection module M2 shown in FIG. 7, the light shielding surface sections can be inclined so as to form an angle of 5 degrees or less on the opposite side of the open portion 89 with respect to the x direction. It is sufficient that the light shielding surface sections are inclined so as to form an angle of 3 degrees or less on the opposite side of the open portion 89 with respect to the x direction.

In the regulating section, the length L of each light shielding surface section in the x direction (first direction) and / or the distance D in the y direction between the two adjacent light shielding surface sections need not be uniform. For example, the light shielding surface sections can be provided close to the open portions 69, 89 (that is, the distance D can be small), or the light shielding surface sections can be provided away from a location of the bottom 101a (that is, the distance D can be large).

In the first detection module M1, another regulating section can be provided in at least one location among inside another opening arranged facing the light emitting element, between the another opening and the light emitting element, or on the running area side of the another opening. In such a configuration, the another regulating section can include a plurality of another light shielding surface sections that extends along the direction of an optical axis of the light emitting element, and another transparent section that is formed between the another light shielding surface sections and allows the light emitted from the light emitting element to pass through. By applying the another regulating section to the light emitting element, only the light that is near the parallel light from the light irradiated toward the yarn can be extracted. For example, even when a light emitting element that does not have a sufficient directivity is applied as a light source, performance of the detecting section is maintained.

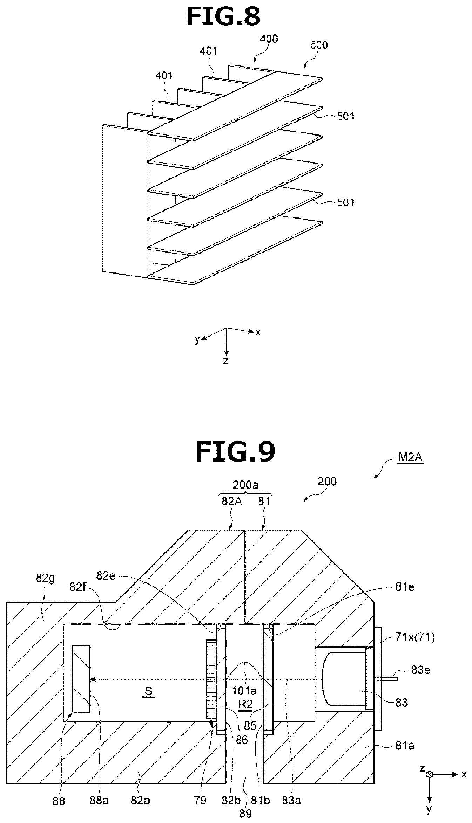

Moreover, as shown in FIG. 8, the yarn monitoring device can include an additional regulating section 500 that overlaps with the regulating section 400 so as to be near in the x direction. The regulating section 400 includes, for example, a plurality of parallel light shielding surface sections 401 that is arranged orthogonal to the y direction. The additional regulating section 500 includes, for example, a plurality of parallel light shielding surface sections (additional light shielding surface sections) 501 that is arranged orthogonal to the z direction. In other words, the additional regulating section 500 can include the plurality of the additional light shielding surface sections in which each additional light shielding surface section forms an angle that is greater than 0 (preferably greater than 45 degrees) but equal to or less than 90 degrees (90 degrees in the present embodiment) on an opposite side of an open portion in the z direction with respect to the light receiving surface, and an additional transparent section (additional passing section) that is formed between the additional light shielding surface sections and allows the transmitted light that passes through the yarn in the running area or the reflected light that is reflected by the yarn to pass through. Each additional light shielding surface section forms an angle with the respective light shielding surface sections. The light shielding surface section 401 shields a disturbance light that is coming from the y direction, and the additional light shielding surface section 501 shields a disturbance light that is coming from the z direction. According to such a configuration, a direction in which the received light is regulated (light shielding direction) by the overlapping regulating section 400 and the additional regulating section 500 can be a combination of any two directions. Accordingly, a disturbance light that is coming from a plurality of the directions (for example, the y direction and the z direction) can be shielded.

Furthermore, the light shielding surface section 401 can be extended in a direction that intersects the y direction. In other words, the light shielding surface section 401 can form an angle of less than 90 degrees with respect to the y direction. The additional light shielding surface section 501 can be extended in a direction that intersects the z direction. In other words, the additional light shielding surface section 501 can form an angle of less than 90 degrees with respect to the z direction. Moreover, the regulating section 400 and the additional regulating section 500 may not be near each other in the x direction. A gap in the x direction can be provided between the regulating section 400 and the additional regulating section 500.

A second holder 200 shown in FIG. 9 can be applied, instead of the second holder 56 (see FIG. 7). Alternatively, the second detection module M2A can be applied. The second holder 200 constitutes the second detection module M2A through which the running yarn Y is passed to detect the state of the yarn Y. A function of the second detection module M2A is the same as that of the second detection module M2 explained above. The light emitting element 83 and the light receiving element 88 that measure a state of the yarn Y that is running through the running area R2 are mounted inside a casing 200a of the second holder 200. The second holder 200 supports the light emitting element 83 and the light receiving element 88.

The second holder 200 differs from the second holder 56 in that the second holder 200 includes a second member 82A having a protruding member 82g, instead of the second member 82 that has a substantially symmetrical shape and the size as that of the first member 81. The light receiving element 88 is provided inside the protruding member 82g; however, the light receiving element 88 is provided farther away from the running area R2, compared to the light receiving element 88 provided in the second holder 56. In other words, a large space S is provided between the opening 82e (or the light receiving window 86) and the light receiving element 88. The space S is the largest space (space in which no part or component is provided) inside the second holder 200. By arranging the light receiving element 88 in such a manner, a sufficient distance can be secured between the yarn Y (yarn path) and the light receiving surface 88a of the light receiving element 88, and an effect of the disturbance light can be reduced. Moreover, a difference in the optical path length between the light transmitted through the yarn Y and the light reflected by the yarn Y increases as the distance from the yarn Y (yarn path) to the light receiving surface 88a of the light receiving element 88 increases. Therefore, because the reflected light from the yarn Y is attenuated before it reaches the light receiving surface 88a, the transmitted light that passes through the yarn Y can be detected with high accuracy.

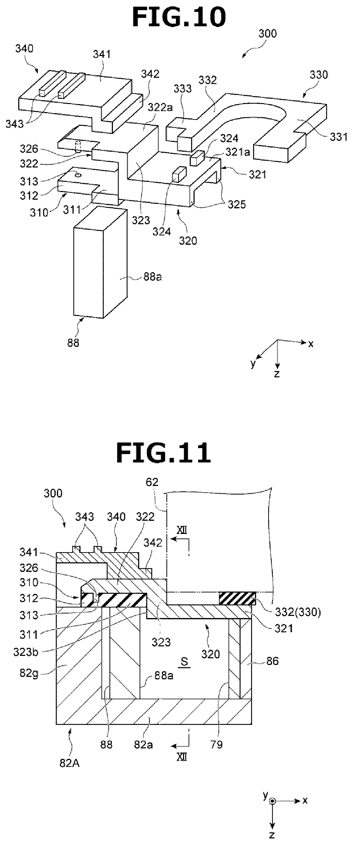

The space S explained above is a part of a rectangular light receiving space 82f formed inside the second member 82A. The light receiving element 88 is arranged in the light receiving space 82f at a location far away from the running area R2. As shown in FIGS. 10 to 12, a dust-proof structure 300 is provided in the second detection module M2A to prevent foreign substance such as fly waste from entering the space S. As shown in FIGS. 10 and 11, the dust-proof structure 300 is provided between the first holder 55 (see FIGS. 3 and 4) and the second holder 200. In other words, the dust-proof structure 300 is provided on one side of the light receiving element 88 in the z direction (downstream side in the running direction of the yarn Y), and closes an upper opening formed on the one side of the second member 82A.

As shown in FIG. 10, the dust-proof structure 300 includes, for example, a first rubber member 310 that contacts the light receiving element 88, a first pressing member 320 that presses the first rubber member 310, a second rubber member 330 that presses a low stepped portion 321 of the first pressing member 320, and a second pressing member 340 that presses a high stepped portion 322 of the first pressing member 320.

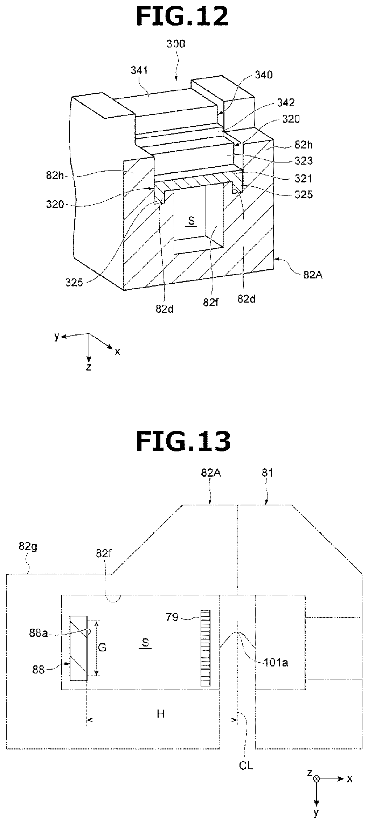

The first rubber member 310 includes a wide portion 311 that presses a part of the light receiving element 88 and a narrowed portion 312 that presses an area between a pair of wiring lines in the light receiving element 88 to avoid a pair of wiring lines from the light receiving element 88 interlinked to the pair of the lead pins 88e (see FIG. 4). As shown in FIG. 12, the first pressing member 320 and the second pressing member 340 are made of, for example, any resin and molded into a predetermined shape. The first pressing member 320 and the second pressing member 340 are accommodated between a pair of positioning members 82h, 82h that are spaced apart in the y direction in the second member 82A. The first pressing member 320 includes the low stepped portion 321 that closes the upper opening of the second member 82A and a high stepped portion 322 that presses the first rubber member 310.

As shown in FIGS. 10 and 11, a stepped portion 323 that extends in the z direction is provided between the low stepped portion 321 and the high stepped portion 322. The stepped portion 323 contacts a back surface 323b of the stepped portion 323 in the wide portion 311 of the first rubber member 310. As shown in FIG. 12, the low stepped portion 321 includes a pair of protruding edge portions 325, 325 that are fitted into a pair of grooved sections 82d, 82d formed along the positioning members 82h, 82h. A labyrinth structure is formed by these protruding edge portions 325 and the grooved sections 82d. The labyrinth structure and the first rubber member 310 shield a path through which foreign substance such as fly waste enters the space S.

A notch having a shape of English letter U corresponding to the running area R2 in a plan view is formed on the second rubber member 330. The second rubber member 330 includes a light emitting side pressing portion 331 that contacts the first member 81, and a light receiving side pressing portion 332 that contacts the second member 82A. The second rubber member 330 is fixed by being pressed by the casing 55a (see FIG. 4) of the first detection module M1. The light receiving side pressing portion 332 that contacts the low stepped portion 321 from above is pressed by the second member 62 (see FIG. 11). As shown in FIG. 10, a protruding member 333 is provided in the light receiving side pressing portion 332, and a pair of protrusions 324, 324 that are spaced apart in the y direction is provided on the low stepped portion 321 of the first pressing member 320. The light receiving side pressing portion 332 that includes the protruding member 333 contacts an upper surface 321a of the low stepped portion 321. The second rubber member 330 is positioned in the x direction and the y direction by fitting the protruding member 333 of the second rubber member 330 between the pair of the protrusions 324, 324.

As shown in FIGS. 10 and 11, a pin 326 that protrudes downward (in the z direction) is provided in the high stepped portion 322 of the first pressing member 320, and the pin 326 is inserted into a hole 313 that is formed in the narrowed portion 312 of the first rubber member 310. The first rubber member 310 is positioned in the y direction and the x direction by fitting the pin 326 into the hole 313. Moreover, the second pressing member 340 includes a leg portion 342 that contacts an upper surface 322a of the high stepped portion 322, and a pressing plate portion 341 formed by a pair of convex rib portions 343 that extends in the y direction.

The dust-proof structure 300 includes two separate members (rubber bushings), the first rubber member 310 and the second rubber member 330. By pressing the light receiving element 88 and the casing 200a with these two members, erroneous pressing is less likely to occur even when dimensional variation occurs. The dust-proof structure 300, as a whole, functions as a lid or a cover for the casing 200a. Each member of the dust-proof structure 300 is positioned in each of the x, y, and z directions, and prevention of fly waste from entering is achieved by using the labyrinth structure.

Dimensions and arrangement of the light receiving element 88 inside the second member 82A (inside the second holder 200) will be explained with reference to FIG. 13. An effective surface width G in the y direction can be defined on the light receiving surface 88a of the light receiving element 88 because it is covered by a part of the surrounding members and the like. The effective surface width G occupies, for example, 80 percent or more, or 90 percent or more of the entire light receiving surface 88a. A size of the light receiving surface 88a of the light receiving element 88 can be easily recognized by a person skilled in the art.

A position of the yarn path in the x direction, that is, a distance H from a center line CL (central plane) that passes through the bottom 101a of the grooved section 101 to the light receiving surface 88a can be defined in the casing 200a. A ratio of the distance H to the effective surface width G can be within the range of 0.5 to 5.0 including both the values. Alternatively, it is sufficient that the ratio of the distance H to the effective surface width G is within the range of 1.0 to 2.5 including both the values.

According to the second detection module M2A explained above, a distance that is sufficient to attenuate the reflected signal from the yarn Y (distance H explained above) is secured between the yarn Y to be measured and the light receiving element 88. Accordingly, a noise component that includes a reflected component from the yarn Y in the optical-type yarn monitoring device 8 (particularly, a signal other than the thickness component of the yarn Y) can be eliminated.

A regulating section that includes the regulating section 400 and the additional regulating section 500 shown in FIG. 8 can be used, instead of the regulating section 79 of the second detection module M2A.

In the second detection module M2, another regulating section can be provided in at least one location among inside the opening 81e formed in the first member 81 in which the light emitting element 83 is provided between the opening 81e and the light receiving surface 88a or on the running area R2 side of the opening 81e. In such a configuration, another regulating section includes a plurality of another light shielding surface sections that extends along the direction of the optical axis 83a of the light emitting element 83, and another transparent section that is formed between the another light shielding surface sections and allows the light emitted from the light emitting element 83 to pass through. By applying another regulating section to the light emitting element 83, only the light that is near the parallel light from the light irradiated toward the yarn Y can be extracted.

The configuration explained in the above embodiments includes the first holder 55 and the second holder 56; however, the configuration can include only one of the first holder 55 and the second holder 56, or can include three or more holders. A sequence in which the first holder 55 and the second holder 56 are arranged is not limited, and the first holder 55 can be arranged on an upstream side of the second holder 56.

In the above embodiments, each of the first detection module M1 and the second detection module M2 can have, for example, a function to detect a running speed of the yarn Y, a function to detect a running length of the yarn Y, and the like, instead of or in addition to the foreign substance detection function and the yarn thickness detection function. The yarn thickness detection function of the second detection module M2 includes not only optical detection of the apparent thickness of the yarn Y but also the mass detection of the yarn Y by using an electrostatic capacitance. For example, the yarn monitoring device according to the present invention can be a yarn monitoring device that combines an optical-type detection module and a capacitive-type detection module. In the optical-type detection module, the configuration (regulating portion and the like) explained in the present invention is applied. Moreover, the yarn monitoring device according to the present invention can detect, as the state of the yarn Y, whether or not the yarn Y is present in the running area and / or whether the yarn Y is running or stationary.

The air spinning device 7, to prevent the twist of the fiber bundle F from being conveyed to the upstream of the air spinning device 7, can further include a needle held by a fiber guiding section and projecting in a spinning chamber. The air spinning device 7 can prevent the twist of the fiber bundle F from being conveyed to the upstream of the air spinning device 7, instead of using the needle, by using a downstream end portion of the fiber guiding section. Furthermore, in the air spinning device 7, instead of the above configuration, a pair of air jet nozzles that twist the fiber bundle F in mutually opposite directions can be arranged.

In the spinning unit 2, the yarn accumulating device 11 is used to pull the yarn Y from the air spinning device 7; however, a delivery roller and a nip roller can be used to pull the yarn Y from the air spinning device 7. In a configuration in which the yarn Y is pulled from the air spinning device 7 with the delivery roller and the nip roller, instead of the yarn accumulating device 11, a slack tube that absorbs the slack of the yarn Y by using a suction airflow, a mechanical compensator, and the like can be provided.

In the spinning machine 1, various devices are arranged such that the yarn Y supplied from the upper side is wound on the lower side in a height direction of the machine frame. However, those devices can be arranged such that the yarn Y supplied from the lower side is wound on the upper side.

In the spinning machine 1, a traversing guide and at least one bottom roller of the drafting device 6 are driven by driving power supplied from the second-end frame 5 (i.e., the driving power is shared among a plurality of the spinning units 2). However, each component (e.g., the drafting device 6, the air spinning device 7, the winding device 13, and the like) of each spinning unit 2 can be driven independently in each spinning unit 2.

The tension sensor 9 can be arranged upstream of the yarn monitoring device 8 in the running direction of the yarn Y. One unit controller 10 can be arranged for each spinning unit 2. Moreover, in the spinning unit 2, the waxing device 12, the tension sensor 9, and the yarn monitoring device 8 can be omitted.

In FIG. 1, the spinning machine 1 is shown to wind the yarn Y into a cheese-shaped package P; however, it is possible to wind the yarn Y into a cone-shaped package P. The yarn Y may slacken when traversing the yarn Y for winding into the cone-shaped package P; however, such slack can be absorbed by the yarn accumulating device 11. The material and shape of various components are not limited to those mentioned above, and it is possible to adopt various materials and shapes. The yarn winding machine according to the present invention is not limited to the spinning machine 1 but can be an automatic winder that is constituted by, for example, a plurality of winder units. Moreover, the yarn monitoring device according to the present invention can be mounted in a textile machine other than a yarn winding machine, which requires monitoring of the state of a running yarn.

A yarn monitoring device according to one aspect of the present invention includes a casing; a detecting section; and a regulating section. The casing includes a first member and a second member having surfaces thereof facing each other in a first direction that is orthogonal to a running direction of a yarn. A running area of the yarn is formed between the first member and the second member, and an open portion that opens toward a second direction that is orthogonal to the running direction and the first direction, and opens toward the running direction is formed. The detecting section detects the yarn that is running inside the running area. The detecting section includes at least one light emitting element provided in at least one of the first member and the second member, and at least one light receiving element provided in at least one of the first member and the second member and including a light receiving surface that is arranged facing an opening formed on the first member and / or the second member. The regulating section includes a plurality of light shielding surface sections that forms an angle within a range of 0 degrees to 90 degrees, including both the values, on an opposite side of the open portion with respect to the light receiving surface, and a passing section that is formed between the light shielding surface sections and allows passage of a transmitted light passed through the yarn running inside the running area or a reflected light reflected by the yarn. The regulating section is provided in at least one location among inside the opening, between the opening and the light receiving surface, and on the running area side of the opening.

According to the present yarn monitoring device, the light receiving element inputs the transmitted light transmitted through the yarn or the reflected light reflected by the yarn via the opening. The light input to the light receiving element passes through the passing section of the regulating section and then reaches the light receiving surface. The regulating section includes the plurality of the light shielding surface sections in which each light shielding surface section forms the angle of 90 degrees or less on the opposite side of the open portion with respect to the light receiving surface. The term "forms the angle" refers to a predetermined angle of higher than 0 degrees formed between the light receiving surface and the light shielding surface section. Because the light shielding surface sections are not parallel to the light receiving surface and each forms an angle on the opposite side of the open portion, it allows the light to pass through the passing section while shielding the incidence of the light from a predetermined direction (for example, the light that enters through the open portion). According to the yarn monitoring device that includes the regulating section, an effect of a disturbance light can be reduced.

In the above yarn monitoring device, the light shielding surface sections can extend in a direction that intersects the second direction and that is parallel to the running direction of the yarn. According to the present configuration, because the light shielding surface sections are not parallel to the second direction, the light shielding surface sections can shield the disturbance light coming from the second direction. The light that enters through the open portion can be shielded.

In the above yarn monitoring device, the light emitting element and the light receiving element can be arranged facing each other in the first direction, and an extending direction of the light shielding surface sections can be the same as a connecting direction that connects the light emitting element and the light receiving element, or can form an angle of 5 degrees or less with respect to the connecting direction. According to the present configuration, the light input to the light receiving element easily passes through the passing section of the regulating section.

In the above yarn monitoring device, the light shielding surface section can have a length (L) in the first direction and the two adjacent light shielding surface sections can be arranged at a distance (D) in the second direction, and a relation between the distance (D) and the length (L), that is, the distance (D)/the length (L) ≤ 1 can hold true. According to the present configuration, the distance (D) is equal to or less than the length (L) (that is, a thickness of the regulating section). Accordingly, the disturbance light from the second direction can be certainly shielded in the yarn monitoring device in which the light emitting element and the light receiving element are arranged facing each other in the first direction.

A ratio of a distance from a position of a yarn path in the first direction to the light receiving surface with respect to an effective surface width in the second direction of the light receiving surface can be within a range of 0.5 to 5.0 including both the values. According to the present configuration, a distance that is sufficient to attenuate the reflected signal from the yarn is secured between the yarn to be measured and the light receiving element. Accordingly, a noise component that includes a reflected component from the yarn in the optical-type yarn monitoring device can be eliminated.

The ratio of the distance from the position of the yarn path in the first direction to the light receiving surface with respect to the effective surface width in the second direction of the light receiving surface can be within a range of 1.0 to 2.5 including both the values. According to the present configuration, the noise component removing effect explained above can be demonstrated more effectively.

In the above yarn monitoring device, the first member can include the light emitting element and the light receiving element, and the light emitting element can be provided on an opposite side of the open portion in the second direction with respect to the light receiving element. According to the present configuration, the effect of the disturbance light can be reduced in a reflected light detecting-type yarn monitoring device.

In the above yarn monitoring device, the light receiving element can include a first light receiving element that includes a first light receiving surface that faces toward a first opening formed on the first member; and a second light receiving element that includes a second light receiving surface that faces toward a second opening formed on the second member. The light emitting element can include a first light emitting element provided in the first member; and a second light emitting element provided in the second member. The regulating section can include a first regulating section provided in the first member; and a second regulating section provided in the second member. The first regulating section can be provided in at least one location among inside the first opening, between the first opening and the first light receiving surface, and on the running area side of the first opening, and the second regulating section can be provided in at least one location among inside the second opening, between the second opening and the second light receiving surface, and the running area side of the second opening. An extending direction of the light shielding surface section of the first regulating section can be the same as a first connecting direction that connects the second light emitting element and the first light receiving element, or can form an angle of 5 degrees or less with respect to the first connecting direction, and an extending direction of the light shielding surface section of the second regulating section can be the same as a second connecting direction that connects the first light emitting element and the second light receiving element, or can form an angle of 5 degrees or less with respect to the second connecting direction. According to the present configuration, the transmitted light and the reflected light emitted from each of the two directions can be detected. For example, the yarn monitoring device can accurately detect a foreign substance contained in the yarn.

In the above yarn monitoring device, the light shielding surface section can have a length (L) in the first direction and the two adjacent light shielding surface sections can be arranged at a distance (D) in the second direction. An extending direction of the light shielding surface section can form an angle (α) with respect to the first direction, and a relation between the distance (D) and the length (L), that is, the distance (D)/the length (L) > tan (α) can hold true. By establishing the relation explained above, for example, the transmitted light can be detected by the second light emitting element and the reflected light can be detected by the first light emitting element. Accordingly, the effect of the disturbance light can be reduced.

In the above yarn monitoring device, the light shielding surface section can have a length (L) in the first direction and the two adjacent light shielding surface sections can be arranged at a distance (D) in the second direction, and a relation between the distance (D) and the length (L), that is, the distance (D)/the length (L) > 1 can hold true. By establishing the relation explained above, for example, the transmitted light can be detected by the second light emitting element and the reflected light can be detected by the first light emitting element. Accordingly, the effect of the disturbance light can be reduced.

In the above yarn monitoring device, the light shielding surface section can have a length (L) in the first direction and the two adjacent light shielding surface sections can be arranged at a distance (D) in the second direction, and a relation between the distance (D) and the length (L), that is, the distance (D)/the length (L) ≤ 3 can hold true. According to the present configuration, the distance (D) is set to shield the incident of the disturbance light while allowing the detection of the reflected light. Accordingly, noise in the detection signal is reduced, thus improving the S/N ratio. The S/N ratio refers to a ratio of the detection signal and noise.

In the above yarn monitoring device, the light shielding surface sections can extend in a direction parallel to the second direction, and extend in the first direction or extend in a direction that makes an angle of 5 degrees or less with respect to the first direction. According to the present configuration, the light that enters through an inlet and / or an outlet of the yarn positioned in the running direction of the yarn inside the casing can be shielded.

The above yarn monitoring device can include an additional regulating section arranged side-by-side in the regulating section in the first direction. The additional regulating section can include a plurality of additional light shielding surface sections that forms an angle of 90 degrees or less on an opposite side of the open portion in the running direction of the yarn with respect to the light receiving surface; and an additional passing section that is formed between the additional light shielding surface sections and allows a transmitted light transmitted through the yarn running inside the running area or a reflected light reflected by the yarn, and each additional light shielding surface section can form an angle with respect to each of the light shielding surface sections. According to the present configuration, a direction in which the received light is regulated (light shielding direction) by the overlapping regulating section and the additional regulating section can be a combination of any two directions. Accordingly, a disturbance light that is coming from a plurality of the directions (for example, the second direction and the running direction) can be shielded.

In the above yarn monitoring device, the regulating section can be provided only on the open portion side of the opening. According to the present configuration, the disturbance light can be reduced while preventing attenuation of light from the light emitting element, that is the light source.

In the above yarn monitoring device, the first member includes the light emitting element and the light receiving element, the light emitting element can face toward another opening formed on the first member, another regulating section can be provided in at least one location among inside the another opening, between the another opening and the light emitting element, and on the running area side of the another opening. The another regulating section can include a plurality of another light shielding surface sections that extends along a direction of an optical axis of the light emitting element; and another passing section that is formed between the another light shielding surface sections and allows the light emitted from the light emitting element to pass through. According to the present configuration, by applying the another regulating section to the light emitting element, only the light that is near the parallel light from the light irradiated toward the yarn can be extracted.

### EFFECTS OF THE INVENTION

According to the yarn monitoring device of the present invention, an effect of a disturbance light can be reduced.

Although the invention has been explained with respect to specific embodiments for a complete and clear disclosure, the appended claims are not to be thus limited but are to be construed as embodying all modifications and alternative constructions that may occur to one skilled in the art that fall within the scope of the claims.

## Claims

1. A yarn monitoring device (8) comprising:
a casing (55a, 56a) that includes a first member (61, 81) and a second member (62, 82, 82A) having surfaces thereof facing each other in a first direction that is orthogonal to a running direction of a yarn (Y), wherein
a running area (R1, R2) of the yarn (Y) is formed between the first member (61, 81) and the second member (62, 82, 82A), and
an open portion (69, 89) that opens toward a second direction that is orthogonal to the running direction and the first direction, and opens toward the running direction is formed;
a detecting section that detects the yarn (Y) that is running inside the running area (R1, R2) and includes
at least one light emitting element (63, 64, 83) provided in at least one of the first member (61, 81) and the second member (62, 82, 82A), and
at least one light receiving element (67, 68, 88) provided in at least one of the first member (61, 81) and the second member (62, 82, 82A) and includes a light receiving surface (67a, 68a, 88a) that is arranged facing an opening (61e, 62e, 82e) formed on the first member (61, 81) and / or the second member (62, 82, 82A); **characterized in that** the yarn monitoring device further comprises:
a regulating section (75, 78, 79, 400) that includes
a plurality of light shielding surface sections (76, 401) that forms an angle within a range of 0 degrees to 90 degrees, including both the values, on an opposite side of the open portion (69, 89) with respect to the light receiving surface (67a, 68a, 88a), and
a passing section (77) that is formed between the light shielding surface sections (76, 401) and allows passage of a transmitted light passed through the yarn (Y) running inside the running area (R1, R2) or a reflected light reflected by the yarn (Y), wherein
the regulating section (75, 78, 79, 400) is provided in at least one location among inside the opening (61e, 62e, 82e), between the opening (61e, 62e, 82e) and the light receiving surface (67a, 68a, 88a), and on the running area (R1, R2) side of the opening (61e, 62e, 82e).

2. The yarn monitoring device (8) as claimed in Claim 1, wherein the light shielding surface sections (76, 401) extend in a direction that intersects the second direction and that is parallel to the running direction of the yarn (Y).

3. The yarn monitoring device (8) as claimed in Claim 1 or 2, wherein
the light emitting element (83) and the light receiving element (88) are arranged facing each other in the first direction, and
an extending direction of the light shielding surface sections (76, 401) is the same as a connecting direction that connects the light emitting element (83) and the light receiving element (88), or forms an angle of 5 degrees or less with respect to the connecting direction.

4. The yarn monitoring device (8) as claimed in Claim 3, wherein
the light shielding surface section (76, 401) has a length (L) in the first direction and the two adjacent light shielding surface sections (76, 401) are arranged at a distance (D) in the second direction, and
a relation between the distance (D) and the length (L), that is, the distance (D)/the length (L) ≤ 1 holds true.

5. The yarn monitoring device (8) as claimed in one of Claims 1 to 4, wherein a ratio of a distance from a position of a yarn path in the first direction to the light receiving surface (88a) with respect to an effective surface width in the second direction of the light receiving surface (88a) is within a range of 0.5 to 5.0 including both the values.

6. The yarn monitoring device (8) as claimed in Claim 5, wherein the ratio of the distance from the position of the yarn path in the first direction to the light receiving surface (88a) with respect to the effective surface width in the second direction of the light receiving surface (88a) is within a range of 1.0 to 2.5 including both the values.

7. The yarn monitoring device (8) as claimed in Claim 1 or 2, wherein
the first member (61) includes the light emitting element (63) and the light receiving element (67), and
the light emitting element (63) is provided on an opposite side of the open portion (89) in the second direction with respect to the light receiving element (67).

8. The yarn monitoring device (8) as claimed in Claim 1 or 2, wherein
the light receiving element (67, 68) includes
a first light receiving element (67) that includes a first light receiving surface (67a) that faces toward a first opening (61e) formed on the first member (61); and
a second light receiving element (68) that includes a second light receiving surface (68a) that faces toward a second opening (62e) formed on the second member (62),
the light emitting element (63, 64) includes
a first light emitting element (63) provided in the first member (61); and
a second light emitting element (64) provided in the second member (62),
the regulating section (75, 78) includes
a first regulating section (75) provided in the first member (61); and
a second regulating section (78) provided in the second member (62), wherein
the first regulating section (75) is provided in at least one location among inside the first opening (61e), between the first opening (61e) and the first light receiving surface (67a), and on the running area (R1) side of the first opening (61e), and the second regulating section (78) is provided in at least one location among inside the second opening (62e), between the second opening (62e) and the second light receiving surface (68a), and the running area (R1) side of the second opening (62e), and
an extending direction of the light shielding surface section (76, 401) of the first regulating section (75) is the same as a first connecting direction that connects the second light emitting element (64) and the first light receiving element (67), or forms an angle of 5 degrees or less with respect to the first connecting direction, and an extending direction of the light shielding surface section (76, 401) of the second regulating section (78) is the same as a second connecting direction that connects the first light emitting element (63) and the second light receiving element (68), or forms an angle of 5 degrees or less with respect to the second connecting direction.

9. The yarn monitoring device (8) as claimed in Claim 8, wherein
the light shielding surface section (76, 401) has a length (L) in the first direction and the two adjacent light shielding surface sections (76, 401) are arranged at a distance (D) in the second direction,
an extending direction of the light shielding surface section (76, 401) forms an angle (α) with respect to the first direction, and
a relation between the distance (D) and the length (L), that is, the distance (D)/the length (L) > tan (α) holds true.

10. The yarn monitoring device (8) as claimed in Claim 8 or 9, wherein
the light shielding surface section (76, 401) has a length (L) in the first direction and the two adjacent light shielding surface sections (76, 401) are arranged at a distance (D) in the second direction, and
a relation between the distance (D) and the length (L), that is, the distance (D)/the length (L) > 1 holds true.

11. The yarn monitoring device (8) as claimed in Claim 8 or 9, wherein
the light shielding surface section (76, 401) has a length (L) in the first direction and the two adjacent light shielding surface sections (76, 401) are arranged at a distance (D) in the second direction, and
a relation between the distance (D) and the length (L), that is, the distance (D)/the length (L) ≤ 3 holds true.

12. The yarn monitoring device (8) as claimed in Claim 1, wherein the light shielding surface sections extend in a direction parallel to the second direction, and extend in the first direction or extend in a direction that makes an angle of 5 degrees or less with respect to the first direction.

13. The yarn monitoring device (8) as claimed in one of Claims 1 to 11 comprising:
an additional regulating section (500) arranged side-by-side in the regulating section (400) in the first direction, wherein
the additional regulating section (500) includes
a plurality of additional light shielding surface sections (501) that forms an angle of 90 degrees or less on an opposite side of the open portion in the running direction of the yarn (Y) with respect to the light receiving surface (67a, 68a, 88a); and
an additional passing section that is formed between the additional light shielding surface sections (501) and allows a transmitted light transmitted through the yarn (Y) running inside the running area (R1, R2) or a reflected light reflected by the yarn (Y), and
each additional light shielding surface section (501) forms an angle with respect to each of the light shielding surface sections (401).

14. The yarn monitoring device (8) as claimed in one of Claims 1 to 13, wherein the regulating section (75, 78, 79, 400) is provided only on the open portion (69, 89) side of the opening (61e, 62e, 82e).

15. The yarn monitoring device (8) as claimed in one of Claims 1 to 14, wherein
the first member (61, 81) includes the light emitting element (63, 64, 83) and the light receiving element (67, 68, 88), wherein
the light emitting element (63, 64, 83) faces toward another opening (81e) formed on the first member (61, 81),
another regulating section is provided in at least one location among inside the another opening (81e), between the another opening (81e) and the light emitting element (63, 64, 83), and on the running area (R1, R2) side of the another opening (81e), and
the another regulating section includes
a plurality of another light shielding surface sections that extends along a direction of an optical axis of the light emitting element (63, 64, 83); and
another passing section that is formed between the another light shielding surface sections and allows the light emitted from the light emitting element (63, 64, 83) to pass through.

## Patentansprüche

1. Garnüberwachungsvorrichtung (8), umfassend:
ein Gehäuse (55a, 56a), das ein erstes Element (61, 81) und ein zweites Element (62, 82, 82A) einschließt, deren Oberflächen in einer ersten Richtung, die orthogonal zu einer Laufrichtung eines Garns (Y) ist, einander zugewandt sind, wobei
ein Laufbereich (R1, R2) des Garns (Y) zwischen dem ersten Element (61, 81) und dem zweiten Element (62, 82, 82A) gebildet ist, und
ein offener Abschnitt (69, 89), der sich in Richtung einer zweiten Richtung, die orthogonal zur Laufrichtung und zur ersten Richtung ist, öffnet und sich in Richtung der Laufrichtung öffnet, gebildet ist;
ein Erfassungsabschnitt, der das Garn (Y), das im Inneren des Laufbereichs (R1, R2) läuft, erfasst und Folgendes einschließt
mindestens ein lichtemittierendes Element (63, 64, 83), das in mindestens einem von dem ersten Element (61, 81) und dem zweiten Element (62, 82, 82A) bereitgestellt ist, und
mindestens ein lichtempfangendes Element (67, 68, 88), das in mindestens einem von dem ersten Element (61, 81) und dem zweiten Element (62, 82, 82A) bereitgestellt ist und eine lichtempfangende Oberfläche (67a, 68a, 88a) einschließt, die einer Öffnung (61e, 62e, 82e) zugewandt angeordnet ist, die auf dem ersten Element (61, 81) und/oder dem zweiten Element (62, 82, 82A) gebildet ist; **dadurch gekennzeichnet, dass** die Garnüberwachungsvorrichtung weiter umfasst:
einen Regulierabschnitt (75, 78, 79, 400), der
eine Vielzahl von lichtabschirmenden Oberflächenabschnitten (76, 401) einschließt, die einen Winkel innerhalb eines Bereichs von 0 Grad bis 90 Grad, einschließlich der beiden Werte, auf einer gegenüberliegenden Seite des offenen Abschnitts (69, 89) in Bezug auf die lichtempfangende Oberfläche (67a, 68a, 88a) bilden, und
einen Durchgangsabschnitt (77), der zwischen den lichtabschirmenden Oberflächenabschnitten (76, 401) gebildet ist und den Durchgang von durch das Garn (Y) hindurchgehendem übertragenen Licht, das im Inneren des Laufbereichs (R1, R2) läuft, oder von durch das Garn (Y) reflektiertem Licht ermöglicht, wobei
der Regulierabschnitt (75, 78, 79, 400) an mindestens einer Stelle im Inneren der Öffnung (61e, 62e, 82e), zwischen der Öffnung (61e, 62e, 82e) und der lichtempfangenden Oberfläche (67a, 68a, 88a) und auf der Seite des Laufbereichs (R1, R2) der Öffnung (61e, 62e, 82e), bereitgestellt ist.

2. Garnüberwachungsvorrichtung (8) nach Anspruch 1, wobei sich die lichtabschirmenden Oberflächenabschnitte (76, 401) in einer Richtung erstrecken, die die zweite Richtung schneidet und parallel zur Laufrichtung des Garns (Y) ist.

3. Garnüberwachungsvorrichtung (8) nach Anspruch 1 oder 2, wobei
das lichtemittierende Element (83) und das lichtempfangende Element (88) einander in der ersten Richtung gegenüberliegend angeordnet sind, und
eine Erstreckungsrichtung der lichtabschirmenden Oberflächenabschnitte (76, 401) mit einer Verbindungsrichtung, die das lichtemittierende Element (83) und das lichtempfangende Element (88) verbindet, übereinstimmt oder einen Winkel von 5 Grad oder weniger in Bezug auf die Verbindungsrichtung bildet.

4. Garnüberwachungsvorrichtung (8) nach Anspruch 3, wobei
der lichtabschirmende Oberflächenabschnitt (76, 401) eine Länge (L) in der ersten Richtung aufweist und die beiden benachbarten lichtabschirmenden Oberflächenabschnitte (76, 401) in einem Abstand (D) in der zweiten Richtung angeordnet sind, und
eine Beziehung zwischen dem Abstand (D) und der Länge (L), das heißt, Abstand (D)/Länge (L) ≤ 1, gilt.

5. Garnüberwachungsvorrichtung (8) nach einem der Ansprüche 1 bis 4, wobei ein Verhältnis eines Abstands von einer Position eines Garnwegs in der ersten Richtung zur lichtempfangenden Oberfläche (88a) in Bezug auf eine effektive Oberflächenbreite in der zweiten Richtung der lichtempfangenden Oberfläche (88a) innerhalb eines Bereichs von 0,5 bis 5,0, einschließlich der beiden Werte, liegt.

6. Garnüberwachungsvorrichtung (8) nach Anspruch 5, wobei das Verhältnis des Abstands von der Position des Garnwegs in der ersten Richtung zur lichtempfangenden Oberfläche (88a) in Bezug auf die effektive Oberflächenbreite in der zweiten Richtung der lichtempfangenden Oberfläche (88a) innerhalb eines Bereichs von 1,0 bis 2,5, einschließlich der beiden Werte, liegt.

7. Garnüberwachungsvorrichtung (8) nach Anspruch 1 oder 2, wobei
das erste Element (61) das lichtemittierende Element (63) und das lichtempfangende Element (67) einschließt, und
das lichtemittierende Element (63) auf einer gegenüberliegenden Seite des offenen Abschnitts (89) in der zweiten Richtung in Bezug auf das lichtempfangende Element (67) bereitgestellt ist.

8. Garnüberwachungsvorrichtung (8) nach Anspruch 1 oder 2, wobei
das lichtempfangende Element (67, 68) Folgendes einschließt
ein erstes lichtempfangendes Element (67), das eine erste lichtempfangende Oberfläche (67a) einschließt, die einer ersten Öffnung (61e) zugewandt ist, die auf dem ersten Element (61) gebildet ist; und
ein zweites lichtempfangendes Element (68), das eine zweite lichtempfangende Oberfläche (68a) einschließt, die einer zweiten Öffnung (62e) zugewandt ist, die auf dem zweiten Element (62) gebildet ist,
das lichtemittierende Element (63, 64) Folgendes einschließt
ein erstes lichtemittierendes Element (63), das in dem ersten Element (61) bereitgestellt ist; und
ein zweites lichtemittierendes Element (64), das in dem zweiten Element (62) bereitgestellt ist,
der Regulierabschnitt (75, 78) Folgendes einschließt
einen ersten Regulierabschnitt (75), der in dem ersten Element (61) bereitgestellt ist; und
einen zweiten Regulierabschnitt (78), der im zweiten Element (62) bereitgestellt ist, wobei
der erste Regulierabschnitt (75) an mindestens einer Stelle im Inneren der ersten Öffnung (61e), zwischen der ersten Öffnung (61e) und der ersten lichtempfangenden Oberfläche (67a) und auf der Seite des Laufbereichs (R1) der ersten Öffnung (61e) bereitgestellt ist, und der zweite Regulierabschnitt (78) an mindestens einer Stelle im Inneren der zweiten Öffnung (62e), zwischen der zweiten Öffnung (62e) und der zweiten lichtempfangenden Oberfläche (68a) und auf der Seite des Laufbereichs (R1) der zweiten Öffnung (62e) bereitgestellt ist, und
eine Erstreckungsrichtung des lichtabschirmenden Oberflächenabschnitts (76, 401) des ersten Regulierabschnitts (75) mit einer ersten Verbindungsrichtung, die das zweite lichtemittierende Element (64) und das erste lichtempfangende Element (67) verbindet, übereinstimmt oder einen Winkel von 5 Grad oder weniger in Bezug auf die erste Verbindungsrichtung bildet, und eine Erstreckungsrichtung des lichtabschirmenden Oberflächenabschnitts (76, 401) des zweiten Regulierabschnitts (78) mit einer zweiten Verbindungsrichtung, die das erste lichtemittierende Element (63) und das zweite lichtempfangende Element (68) verbindet, übereinstimmt oder einen Winkel von 5 Grad oder weniger in Bezug auf die zweite Verbindungsrichtung bildet.

9. Garnüberwachungsvorrichtung (8) nach Anspruch 8, wobei
der lichtabschirmende Oberflächenabschnitt (76, 401) eine Länge (L) in der ersten Richtung aufweist und die beiden benachbarten lichtabschirmenden Oberflächenabschnitte (76, 401) in einem Abstand (D) in der zweiten Richtung angeordnet sind,
eine Erstreckungsrichtung des lichtabschirmenden Oberflächenabschnitts (76, 401) ein Winkel (α) in Bezug auf die erste Richtung bildet, und
eine Beziehung zwischen dem Abstand (D) und der Länge (L), das heißt, Abstand (D)/Länge (L) > tan (α), gilt.

10. Garnüberwachungsvorrichtung (8) nach Anspruch 8 oder 9, wobei
der lichtabschirmende Oberflächenabschnitt (76, 401) eine Länge (L) in der ersten Richtung aufweist und die beiden benachbarten lichtabschirmenden Oberflächenabschnitte (76, 401) in einem Abstand (D) in der zweiten Richtung angeordnet sind, und
eine Beziehung zwischen dem Abstand (D) und der Länge (L), das heißt, Abstand (D)/Länge (L) > 1, gilt.

11. Garnüberwachungsvorrichtung (8) nach Anspruch 8 oder 9, wobei
der lichtabschirmende Oberflächenabschnitt (76, 401) eine Länge (L) in der ersten Richtung aufweist und die beiden benachbarten lichtabschirmenden Oberflächenabschnitte (76, 401) in einem Abstand (D) in der zweiten Richtung angeordnet sind, und
eine Beziehung zwischen dem Abstand (D) und der Länge (L), das heißt, Abstand (D)/Länge (L) ≤ 3, gilt.

12. Garnüberwachungsvorrichtung (8) nach Anspruch 1, wobei sich die lichtabschirmenden Oberflächenabschnitte in einer Richtung parallel zur zweiten Richtung erstrecken und sich in der ersten Richtung oder in einer Richtung erstrecken, die einen Winkel von 5 Grad oder weniger in Bezug auf die erste Richtung bildet.

13. Garnüberwachungsvorrichtung (8) nach einem der Ansprüche 1 bis 11, umfassend:
einen zusätzlichen Regulierabschnitt (500), der in der ersten Richtung im Regulierabschnitt (400) Seite an Seite angeordnet ist, wobei
der zusätzliche Regulierabschnitt (500) Folgendes einschließt
eine Vielzahl von zusätzlichen lichtabschirmenden Oberflächenabschnitten (501), die einen Winkel von 90 Grad oder weniger auf einer gegenüberliegenden Seite des offenen Abschnitts in der Laufrichtung des Garns (Y) in Bezug auf die lichtempfangende Oberfläche (67a, 68a, 88a) bilden; und
einen zusätzlichen Durchgangsabschnitt, der zwischen den zusätzlichen lichtabschirmenden Oberflächenabschnitten (501) gebildet ist und ein übertragenes Licht, das durch das im Inneren des Laufbereichs (R1, R2) laufende Garn (Y) übertragen wird, oder ein vom Garn (Y) reflektiertes Licht durchlässt, und
jeder zusätzliche lichtabschirmende Oberflächenabschnitt (501) einen Winkel in Bezug auf jeden der lichtabschirmenden Oberflächenabschnitte (401) bildet.

14. Garnüberwachungsvorrichtung (8) nach einem der Ansprüche 1 bis 13, wobei der Regulierabschnitt (75, 78, 79, 400) nur auf der Seite des offenen Abschnitts (69, 89) der Öffnung (61e, 62e, 82e) bereitgestellt ist.

15. Garnüberwachungsvorrichtung (8) nach einem der Ansprüche 1 bis 14, wobei
das erste Element (61, 81), das lichtemittierende Element (63, 64, 83) und das lichtempfangende Element (67, 68, 88) einschließt, wobei
das lichtemittierende Element (63, 64, 83) einer anderen Öffnung (81e) zugewandt ist, die auf dem ersten Element (61, 81) gebildet ist,
ein anderer Regulierabschnitt an mindestens einer Stelle im Inneren der anderen Öffnung (81e), zwischen der anderen Öffnung (81e) und dem lichtemittierenden Element (63, 64, 83) und auf der Seite des Laufbereichs (R1, R2) der anderen Öffnung (81e) bereitgestellt ist, und
der andere Regulierabschnitt Folgendes einschließt
eine Vielzahl von anderen lichtabschirmenden Oberflächenabschnitten, die sich entlang einer Richtung einer optischen Achse des lichtemittierenden Elements (63, 64, 83) erstrecken; und
einen anderen Durchgangsabschnitt, der zwischen den anderen lichtabschirmenden Oberflächenabschnitten gebildet ist und das vom lichtempfangenden Element (63, 64, 83) emittierte Licht durchlässt.

## Revendications

1. Dispositif (8) de surveillance de fil comprenant :
un boîtier (55a, 56a) qui inclut un premier élément (61, 81) et un second élément (62, 82, 82A), les surfaces de ceux-ci se faisant mutuellement face dans une première direction qui est orthogonale à une direction de déplacement d'un fil (Y), dans lequel
une zone de déplacement (R1, R2) du fil (Y) est formée entre le premier élément (61, 81) et le second élément (62, 82, 82A), et
une partie ouverte (69, 89) qui s'ouvre vers une seconde direction qui est orthogonale à la direction de déplacement et à la première direction, et s'ouvre vers la direction de déplacement est formée ;
une section de détection qui détecte le fil (Y) qui se déplace à l'intérieur de la zone de déplacement (R1, R2) et inclut
au moins un élément électroluminescent (63, 64, 83) disposé dans au moins l'un du premier élément (61, 81) et du second élément (62, 82, 82A), et
au moins un élément de réception de lumière (67, 68, 88) disposé dans au moins un du premier élément (61, 81) et du second élément (62, 82, 82A) et inclut une première surface de réception de lumière (67a, 68a, 88a) qui est agencée faisant face à une ouverture (61e, 62e, 82e) formée sur le premier élément (61, 81) et/ou le second élément (62, 82, 82A) ; **caractérisé en ce que** le dispositif de surveillance de fil comprend en outre :
une section de régulation (75, 78, 79, 400) qui inclut
une pluralité de sections de surface de protection contre la lumière (76, 401) qui forme un angle dans une plage de 0 degré à 90 degrés, incluant les deux valeurs, sur un côté opposé de la partie ouverte (69, 89) par rapport à la surface de réception de lumière (67a, 68a, 88a), et
une section de passage (77) qui est formée entre les sections de surface de protection contre la lumière (76, 401) et permet le passage d'une lumière transmise ayant traversé le fil (Y) se déplaçant à l'intérieur de la zone de déplacement (R1, R2) ou d'une lumière réfléchie qui est réfléchie par le fil (Y), dans lequel
la section de régulation (75, 78, 79, 400) est disposée dans au moins un emplacement parmi l'intérieur de l'ouverture (61e, 62e, 82e), entre l'ouverture (61e, 62e, 82e) et la surface de réception de lumière (67a, 68a, 88a), et sur le côté zone de déplacement (R1, R2) de l'ouverture (61e, 62e, 82e).

2. Dispositif (8) de surveillance de fil selon la revendication 1, dans lequel les sections de surface de protection contre la lumière (76, 401) s'étendent dans une direction qui coupe la seconde direction et qui est parallèle à la direction de déplacement du fil (Y).

3. Dispositif (8) de surveillance de fil selon la revendication 1 ou revendication 2, dans lequel
l'élément électroluminescent (83) et l'élément de réception de lumière (88) sont agencés se faisant mutuellement face dans la première direction, et
une direction d'extension des sections de surface de protection contre la lumière (76, 401) est la même qu'une direction de liaison qui relie l'élément électroluminescent (83) et l'élément de réception de lumière (88), ou forme un angle de 5 degrés ou moins par rapport à la direction de liaison.

4. Dispositif (8) de surveillance de fil selon la revendication 3, dans lequel
la section de surface de protection contre la lumière (76, 401) présente une longueur (L) dans la première direction et les deux sections de surface de protection contre la lumière (76, 401) adjacentes sont agencées à une distance (D) dans la seconde direction, et
une relation entre la distance (D) et la longueur (L), c'est-à-dire, la distance (D)/la longueur (L) ≤ 1 est vraie.

5. Dispositif (8) de surveillance de fil selon l'une des revendications 1 à 4, dans lequel un rapport d'une distance partant d'une position d'un chemin de fil dans la première direction jusqu'à la surface de réception de lumière (88a) par rapport à une largeur de surface effective dans la seconde direction de la surface de réception de lumière (88a) est dans une plage de 0,5 à 5,0 incluant les deux valeurs.

6. Dispositif (8) de surveillance de fil selon la revendication 5, dans lequel le rapport de la distance partant de la position du chemin de fil dans la première direction jusqu'à la surface de réception de lumière (88a) par rapport à la largeur de surface effective dans la seconde direction de la surface de réception de lumière (88a) est dans une plage de 1,0 à 2,5 incluant les deux valeurs.

7. Dispositif (8) de surveillance de fil selon la revendication 1 ou revendication 2, dans lequel
le premier élément (61) inclut l'élément électroluminescent (63) et l'élément de réception de lumière (67), et
l'élément électroluminescent (63) est disposé sur un côté opposé de la partie ouverte (89) dans la seconde direction par rapport à l'élément de réception de lumière (67).

8. Dispositif (8) de surveillance de fil selon la revendication 1 ou revendication 2, dans lequel
l'élément de réception de lumière (67, 68) inclut
un premier élément de réception de lumière (67) qui inclut une première surface de réception de lumière (67a) qui est tournée vers une première ouverture (61e) formée sur le premier élément (61) ; et
un second élément de réception de lumière (68) qui inclut une seconde surface de réception de lumière (68a) qui est tournée vers une seconde ouverture (62e) formée sur le second élément (62),
l'élément électroluminescent (63, 64) inclut
un premier élément électroluminescent (63) disposé dans le premier élément (61) ; et
un second élément électroluminescent (64) disposé dans le second élément (62),
la section de régulation (75, 78) inclut
une première section de régulation (75) disposée dans le premier élément (61) ; et
une seconde section de régulation (78) disposée dans le second élément (62), dans lequel
la première section de régulation (75) est disposée dans au moins un emplacement parmi l'intérieur de la première ouverture (61e), entre la première ouverture (61e) et la première surface de réception de lumière (67a), et sur le côté zone de déplacement (R1) de la première ouverture (61e), et la seconde section de régulation (78) est disposée dans au moins un emplacement parmi l'intérieur de la seconde ouverture (62e), entre la seconde ouverture (62e) et la seconde surface de réception de lumière (68a), et sur le côté zone de déplacement (R1) de la seconde ouverture (62e),
une direction d'extension de la section de surface de protection contre la lumière (76, 401) de la première section de régulation (75) est la même qu'une première direction de liaison qui relie le second élément électroluminescent (64) et le premier élément de réception de lumière (67), ou forme un angle de 5 degrés ou moins par rapport à la première direction de liaison, et une direction d'extension de la section de surface de protection contre la lumière (76, 401) de la seconde section de régulation (78) est la même qu'une seconde direction de liaison qui relie le premier élément électroluminescent (63) et le second élément de réception de lumière (68), ou forme un angle de 5 degrés ou moins par rapport à la seconde direction de liaison.

9. Dispositif (8) de surveillance de fil selon la revendication 8, dans lequel
la section de surface de protection contre la lumière (76, 401) présente une longueur (L) dans la première direction et les deux sections de surface de protection contre la lumière (76, 401) adjacentes sont agencées à une distance (D) dans la seconde direction,
une direction d'extension de la section de surface de protection contre la lumière (76, 401) forme un angle (α) par rapport à la première direction, et
une relation entre la distance (D) et la longueur (L), c'est-à-dire, la distance (D)/la longueur (L) > tan (α) est vraie.

10. Dispositif (8) de surveillance de fil selon la revendication 8 ou revendication 9, dans lequel
la section de surface de protection contre la lumière (76, 401) présente une longueur (L) dans la première direction et les deux sections de surface de protection contre la lumière (76, 401) adjacentes sont agencées à une distance (D) dans la seconde direction, et
une relation entre la distance (D) et la longueur (L), c'est-à-dire, la distance (D)/la longueur (L) > 1 est vraie.

11. Dispositif (8) de surveillance de fil selon la revendication 8 ou revendication 9, dans lequel
la section de surface de protection contre la lumière (76, 401) présente une longueur (L) dans la première direction et les deux sections de surface de protection contre la lumière (76, 401) adjacentes sont agencées à une distance (D) dans la seconde direction, et
une relation entre la distance (D) et la longueur (L), c'est-à-dire, la distance (D)/la longueur (L) ≤ 3 est vraie.

12. Dispositif (8) de surveillance de fil selon la revendication 1, dans lequel les sections de surface de protection contre la lumière s'étendent dans une direction parallèle à la seconde direction, et s'étendent dans la première direction ou s'étendent dans une direction qui crée un angle de 5 degrés ou moins par rapport à la première direction.

13. Dispositif (8) de surveillance de fil selon l'une des revendications 1 à 11 comprenant :
une section de régulation supplémentaire (500) agencée côte à côte dans la section de régulation (400) dans la première direction, dans lequel
la section de régulation supplémentaire (500) inclut
une pluralité de sections de surface de protection contre la lumière supplémentaires (501) qui forme un angle de 90 degrés ou moins sur un côté opposé de la partie ouverte dans la direction de déplacement du fil (Y) par rapport à la surface de réception de lumière (67a, 68a, 88a) ; et
une section de passage supplémentaire qui est formée entre les sections de surface de protection contre la lumière supplémentaires (501) et permet le passage une lumière transmise qui est transmise à travers le fil (Y) se déplaçant à l'intérieur de la zone de déplacement (R1, R2) ou une lumière réfléchie qui est réfléchie par le fil (Y), et
chaque section de surface de protection contre la lumière supplémentaire (501) forme un angle par rapport à chacune des sections de surface de protection contre la lumière (401).

14. Dispositif (8) de surveillance de fil selon l'une des revendications 1 à 13, dans lequel la section de régulation (75, 78, 79, 400) est disposée uniquement sur le côté partie ouverte (69, 89) de l'ouverture (61e, 62e, 82e).

15. Dispositif (8) de surveillance de fil selon l'une des revendications 1 à 14, dans lequel
le premier élément (61, 81) inclut l'élément électroluminescent (63, 64, 83) et l'élément de réception de lumière (67, 68, 88), dans lequel
l'élément électroluminescent (63, 64, 83) est tourné vers une autre ouverture (81e) formée sur le premier élément (61, 81),
une autre section de régulation est disposée dans au moins un emplacement parmi l'intérieur de l'autre ouverture (81e), entre l'autre ouverture (81e) et l'élément électroluminescent (63, 64, 83), et sur le côté zone de déplacement (R1, R2) de l'autre ouverture (81e), et
l'autre section de régulation inclut
une pluralité d'autres sections de surface de protection contre la lumière qui s'étend le long d'une direction d'un axe optique de l'élément électroluminescent (63, 64, 83) ; et
une autre section de passage qui est formée entre les autres sections de surface de protection contre la lumière et permet à la lumière émise depuis l'élément électroluminescent (63, 64, 83) de traverser.
